# EUROPÄISCHE PATENTSCHRIFT

(11) **EP 0 519 870 B1**
(45) Veröffentlichungstag und Bekanntmachung des Hinweises auf die Patenterteilung: **11.09.1996**
(21) Anmeldenummer: 92810458.7
(22) Anmeldetag: 12.06.1992
(51) Int. Cl.: A61K 31/195, A61K 9/54

(54) **Neue orale Diclofenaczubereitung**
New oral diclofenac composition
Nouvelle composition à base de diclofenac pour l'administration orale

(30) Priorität: 17.06.1991 EP 91810460
(43) Veröffentlichungstag der Anmeldung: 23.12.1992
(73) Patentinhaber: SPIRIG AG PHARMAZEUTISCHE PRÄPARATE, CH-4622 Egerkingen (CH)
(72) Erfinder: Juch, Rolf-Dieter, CH-4612 Wangen b. Olten (CH)
(74) Vertreter: Braun, André, jr.

(56) Entgegenhaltungen:
- US-A- 4 980 170
- PATENT ABSTRACTS OF JAPAN, vol. 11, no. 365 (C-460), 18. Juni 1987; & JP-A-62 135 419

## Beschreibung

Die Erfindung betrifft eine neue Diclofenaczubereitung mit gesteuerter Wirkstofffreisetzung, wie sie in den Patentansprüchen beschrieben wird.

Das nichtsteroide Diclofenac hat sich seit langem wegen seiner starken entzündungshemmenden und schmerzlindernden Wirkung in der Langzeittherapie von rheumatischen Erkrankungen bewährt.

Es ist jedoch allgemein bekannt, dass die Blutkonzentrations-Halbwertszeit von oralen Diclofenaczubereitungen mit einer bis zwei Stunden sehr kurz ist. Deswegen muss das Arzneimittel üblicherweise mindestens dreimal pro Tag verabreicht werden. Die häufige Einnahme des Arzneimittels bewirkt aber eine ungenügende Patienten compliance, zumal mit einer verschlechterten Mitwirkung der Patienten vor allem auch deswegen zu rechnen ist, weil diese häufig fortgeschrittenen Alters sind und täglich mehrere verschiedene Arzneimittel zu sich nehmen müssen. Dies erschwert jedoch eine klinische Kontrolle und den Therapieerfolg.

Problematisch können zudem die durch den raschen Wirkstoffanstieg im Blut bewirkten Nebeneffekte, wie z.B. Magen-Darmbeschwerden verschiedenster Art, sein. Da die therapeutische Behandlung einer rheumatischen Entzündung immer einen Kompromiss zwischen einer erfolgreichen Bekämpfung der Symptome, was einen ausreichenden Blutplasmaspiegel von Diclofenac erfordert, und einem Akzeptieren unerwünschter Nebenwirkungen, was bekanntlich durch überflüssig hohe Blutplasmawerte entscheidend beeinflusst wird, darstellt, hat man schon versucht, diese Nebeneffekte durch die Beeinflussung der Freisetzung von Diclofenac zu steuern.

Es ist bekannt, dass Diclofenac sich in der Synovialflüssigkeit mit Verzögerung aus dem Blutplasma anreichert. Die Austauschvorgänge Blutplasma-Synovialflüssigkeit verlaufen im Verhältnis zur Plasmakinetik langsam. Es ist deshalb nicht notwendig, einen hohen Blutplasmaspiegel über viele Stunden zu halten. Weil die Eliminationshalbwertszeit von nicht retardiertem Diclofenac in einem Bereich von 1-2 Stunden liegt, reicht jedoch in der Regel eine zweimalige orale Applikation am Tag nicht aus. So erwähnt Fenner in Tempo Medical (APV-Kurs über Antirheumatika, Nürnberg 1983), dass selbst retardierte Formen von Diclofenac nur bedingt geeignet sind, den durch die kurzen Blutplasma eliminationshalbwertszeiten bedingten Plasmaspiegelverlauf so zu beeinflussen, dass auch noch nach 8 bis 10 Stunden eine nennenswerte Steuerung des Blutplasmaspiegels erfolgen kann, weil die Retardierung nach diesem Zeitraum zu einer starken Abflachung der Blutplasmaspiegelkurve führt.

Dem Fachmann stellt sich bei der Entwicklung neuer galenischer Verabreichungsformen deshalb immer das Problem, den Blutplasmaspiegel von Diclofenac einerseits so hoch zu halten, dass die Wirkung auch über Nacht anhält und so beispielsweise Symptome wie Morgensteifigkeit vermieden werden können, und andererseits die Wirkung von Diclofenac schon kurz nach der Einnahme eintritt.

Zur Erreichung dieses Ziels wird häufig empfohlen, die Einnahme eines magensaftresistenten Dragées mit der Verabreichung eines Suppositoriums oder Retard-Dragées vor dem Schlafengehen zu kombinieren. Dies kann für den Patienten zu folgenschweren Verwechslungen mit erhöhten Nebenwirkungen oder mangelhafter kurzfristiger Schmerzlinderung führen.

Um dies zu vermeiden, hat man versucht, beide Wirkungsarten von Diclofenac in einer einzigen Verabreichungsform zu kombinieren.

In der JP 61/44811 (Veröffentlichungsdatum 4. März 1986) wird eine Kombination eines initial-freisetzenden Granulatgemisches mit verzögert freisetzendem Wirkstoffanteil beansprucht. Da der Wirkstoff im initial-freisetzenden Granulatanteil schon im Magen freigesetzt wird, ist wegen der schleimhautreizenden resp. -schädigenden Wirkung von Diclofenac mit nachteiligen Nebenwirkungen zu rechnen.

Der Diclofenac verzögert freisetzende Granulatanteil ist mit einer Hülle enthaltend Methacrylsäure-Methylmethacrylat-Copolymer beschichtet, die sich in Wasser bei einem pH von 6-7 löst. Diese Hülle neigt bei der Beschichtung und Trocknung zum Bruch. Die Bruchstücke weisen eine Tendenz zum Agglomerieren auf. Dadurch wird einerseits die Ausbeute an Partikeln der gewünschten Grösse vermindert und andererseits die verzögert-freisetzende Wirkung dieser Partikel aufgehoben.

Ein weiterer Entwicklungsschritt bei Diclofenacformulierungen besteht darin, obige Nachteile zu beheben. So wird in EP 348 808 ein pelletiertes orales Diclofenacpräparat beschrieben, dessen Wirkstoff zu einem Teil in retardiert freisetzender und zu einem andern Teil in magensaftresistenter Form vorliegt. Erreicht wird dieses Ziel durch Umhüllung eines Teils der Pellets mit einer für Diclofenac retardiert durchlässigen Diffusionsmembran und eines andern Teils der Pellets mit einer magensaftresistenten Membran. Die Diffusionsmembran besteht dabei aus mindestens einer Schicht eines Acrylharzes. Da Diclofenac Säurecharakter mit einem pKa-Wert von ca. 4-5 aufweist, nimmt dessen Grundlöslichkeit im wässrigen Medium oberhalb von diesem Wert wesentlich zu. Damit nimmt in der Regel auch die Wechselwirkung mit den Polymeren, die für die magensaftresistente Umhüllung von Pellets verwendet werden, zu. Bei gleichzeitiger Diclofenaceinnahme und Nahrungsaufnahme kann dann bei einer dadurch bedingten Erhöhung des pH-Werts bis ca. 5 im Magen der Wirkstoff durchaus schon freigesetzt werden. Um dies zu verhindern, wird eine zweischichtige magensaftresistente Membran aus Celluloseether mit unterschiedlichem Substitutionsgrad, die mit Phthalsäureanhydrid verestert ist, vorgeschlagen, wobei in deren inneren Schicht als Hilfsstoff eine wasserunlösliche organische Säure eingearbeitet wird.

Ziel der in der EP 383 967 beschriebenen Erfindung ist es, ein pelletiertes orales Diclofenacpräparat mit verlängerter Wirkung und verbesserter magensaftresistenter Hülle bereitzustellen, so dass die Partikel nicht mehr agglomerieren.

Gelöst wird das Problem durch ein Präparat mit einem Anteil an schnell-freisetzendem Diclofenac und einem Teil magensaftresistent umhüllter Pellets. Die Hülle besteht aus 100 Teilen Methacrylsäure-Methylmethacrylat-Copolymer, 3-40 Teilen Glycerinfettsäureester und 1-150 Teilen Talcum.

Auch in der DE-OS 39 15 150 wird ein langwirkendes Diclofenacpräparat beansprucht, das eine Komponente mit rascher Wirkstofffreigabe und eine mit verzögerter Wirkstofffreigabe umfasst. Dabei wird die Diclofenac mit verzögerter Freigabe enthaltende Komponente mit einer organischen Säure vermischt und das Gemisch mit einer üblichen Hülle versehen.

Bei den vorerwähnten Kombinationspräparaten wird jeweils ein schnell-freisetzendes und verzögert-freisetzendes Diclofenac miteinander gemischt und dann meist zu Tabletten gepresst oder in Kapseln gefüllt. Eine quantitativ exakte Mischung herzustellen, ist jedoch kompliziert. Das Abfüllen zweier Komponenten in eine Kapsel ist aber aus einer Mischung wegen möglicher Inhomogenitäten problematisch; eine getrennte Abfüllung zweier Komponenten ist technisch aufwendiger und bei derart kleinen Füllmengen wegen der erforderlichen Dosierungsgenauigkeit nicht ratsam.

Da schon kleine Schwankungen in der quantitativen Zusammensetzung oder nicht homogenes Vermischen der Komponenten eines Diclofenacpräparats bedeutende Schwankungen im Blutplasmawert hervorrufen können, wäre es von grossem Vorteil, ein Diclofenacpräparat mit gesteuerter Wirkstofffreisetzung, das nur aus einer einzigen Komponente besteht, bereitzustellen.

Aufgabe der vorliegenden Erfindung ist es deshalb, eine Diclofenac-haltige orale Zubereitung mit gesteuerter Wirkstofffreisetzung zur Verfügung zu stellen, bei dessen Einnahme möglichst sofort eine Schmerzlinderung eintritt und diese über einen längeren Zeitraum von ca. 12 Stunden erhalten bleibt. In dieser Schrift bedeutet Diclofenac die freie Säure und deren Salze, insbesondere Diclofenac-Na.

Weitere Aufgaben der Erfindung bestehen darin, ein Höchstmass an Reproduzierbarkeit für die Freisetzung des Wirkstoffs zu erzielen, keine organischen Lösungsmittel beim Aufbau des Präparats zu verwenden, um das Verbleiben von Restlösemittel im Endprodukt zu vermeiden, und eine mehrjährige Stabilität in der Freisetzungscharakteristik bei normaler Lagerung der Zubereitung zu gewährleisten.

Gelöst wurden diese Aufgaben erfindungsgemäss durch eine Diclofenac-haltige pelletierte orale Arzneimittelzubereitung mit gesteuerter Wirkstofffreisetzung, die eine auf einem Neutralpellet aufgetragene Wirkstoffschicht, eine vorzugsweise 10-20 Gew.-% der Endpellets betragende innere Membranschicht, wobei 35-65 Gew.-%, vorzugsweise 45-55 Gew.-%, der Membranschicht aus mindestens einem wasserunlöslichen Polymer, 5-20 Gew.-%, vorzugsweise 6-13 Gew.-%, aus mindestens einem Porenbildner und 20-50 Gew.-%, vorzugsweise 25-46 Gew.-%, aus Hilfsstoffen besteht, und eine äussere magensaftresistente Lackschicht enthält.

Bevorzugterweise wird die erfindungsgemässe Arzneimittelzubereitung in Pelletform in eine Kapsel, besonders bevorzugt in eine Hartgelatinekapsel, eingeschlossen.

Durch die erfindungsgemässe multiple-unit Arzneimittelform werden die bisher vorhandenen Probleme bei der Herstellung und Verabreichung von Diclofenacpräparaten weitgehend gelöst.

Die Wirkstoffschicht enthält den Wirkstoff und Hilfsstoffe, die erfindungsgemäss auf handelsübliche Neutralpellets (Nonpareils) aufgetragen werden.

Geeignete Korngrössen der Neutralpellets weisen einen Durchmesser von 0,5 mm bis 1,2 mm, vorzugsweise 0,6 mm bis 0,85 mm mit weniger als 5 Gew.-% Über- resp. Untergrössen, auf. Der Aufbau mit Diclofenac erfolgt dann mit üblichen Auftragstechniken, z.B. in rotierenden Kesseln, vorzugsweise aber in Wirbeischichtsprühanlagen, die vorzugsweise mit einem Wurster-Einsatz oder Rotorprozessor-Einsatz bestückt sind.

Die Zugabe von Diclofenac erfolgt vorzugsweise in einer Suspension in Wasser, die als Hilfsstoffe vorzugsweise Bindemittel, wie Povidon oder Cellulosederivate, wie z.B. Hydroxypropylcellulose oder Hydroxypropylmethylcellulose, Weichmacher, wie Macrogol®, und Gleit- resp. Trennmittel, wie Talcum, Mg-Stearat, Syloid® oder Aerosil®, und gegebenenfalls eine Silicon-Antischaumemulsion enthält. Der Suspension kann als "Indikator" für die Überprüfung der Magensaftresistenz gemäss CH-Patent 656 310 z.B. Nicotinamid zugesetzt werden.

Als besonders bevorzugte Hilfsstoffe wird ein Gemisch, das Povidone® K 30, Macrogol® 400 und Talcum oder Mg-stearat und gegebenenfalls ein Silicon-Antischaummittel und/oder als Indikator Nicotinamid enthält, verwendet.

Die Diclofenac enthaltende Suspension wird vorzugsweise durch Aufsprühen auf die Neutralpellets aufgetragen. Die derart mit der Wirkstoffschicht beladenen Neutralpellets werden bis auf eine Restfeuchte von 0,1-4,0 Gew.-%, vorzugsweise 0,4-1,0 Gew.-%, jeweils bezogen auf die unbeschichteten Pellets, getrocknet. Der Aufbau der Wirkstoffschicht ist beendet, wenn die Neutralpellets um 110-160 Gew.-%, vorzugsweise 130-140 Gew.-%, schwerer geworden sind. Solcherweise werden vorzugsweise Pellets mit einem Wirkstoffanteil von 20-45 Gew.-% und 1-15 Gew.-% Hilfsstoffe, bezogen auf die Endpellets (als Endpellets werden die erfindungsgemäss zweifach beschichteten Pellets bezeichnet), hergestellt.

Durch Siebung der Pellets kann man die für die Reproduzierbarkeit der Freisetzung geeignete Pelletgrösse im Bereich von 0,6 mm bis 1,4 mm, vorzugsweise 0,8 mm bis 1,25 mm, aussortieren.

Die angestrebte Wirkstofffreisetzung wird insbesondere bestimmt durch den Aufbau der inneren Membranschicht und der äusseren Lackschicht.

Die innere Membranschicht steuert die Diffusion von Diclofenac aus dem Innern des Pellets in die umgebende Flüssigkeit. Zur Steuerung der Diffusionscharakteristik und zur Gewährleistung der gewünschten Freisetzung von Diclofenac ist es notwendig, dass diese Schicht im Laufe der Diffusion und Wirkstoffverarmung des Pellets nicht platzt oder sich auflöst, sondern nur schrumpft, den Wirkstoff aber gleichwohl in der gewünschten Menge freisetzt.

Erreicht wird dieses Ziel durch die Applikation eines Gemisches, das mindestens ein geeignetes wasserunlösliches Polymer, wie z.B. wasserunlösliche Acrylharze, z.B. Acrylsäure-Methacrylestercopolymere, wie sie unter der Bezeichnung Eudragit® RL 30 D, Eudragit® RS 30 D oder Eudragit® NE 30 D von Röhm, Darmstadt, Deutschland, vertrieben werden, Surelease® oder Surelease® XM in einer Menge von 35-65 Gew.-%, vorzugsweise 45-55 Gew.-%, mindestens einen geeigneten wasserlöslichen oder wasserunlöslichen Porenbildner oder ein Gemisch davon in einer Menge von 5-20 Gew.-%, vorzugsweise 6-13 Gew.-%, und Hilfsstoffe, wie z.B. Weichmacher, wie Triacetin oder Diethylphthalat, oder Gleitmittel, wie z.B. Talcum, Mg-stearat, Syloid® oder Aerosil®, und gegebenenfalls Antischaumemulsionen, vorzugsweise eine Silicon-Antischaumemulsion, in einer Menge von 20-50 Gew.-%, vorzugsweise 25-45 Gew.-%, jeweils bezogen auf die Membranschicht, enthält.

Als wasserunlösliche Porenbildner können z.B. Kaolin, Calciumcarbonat, Calciumhydrogenphosphat, Magnesiumoxid, mikrokristalline Cellulose, Titandioxid oder Eisenoxid verwendet werden.

Als wasserlösliche Porenbildner können z.B. Povidone® K 30, Polyvinylalkohol, Cellulosederivate, wie Hydroxypropylcellulose, Hydroxypropylmethylcellulose, Methylcellulose oder Natriumcarboxymethylcellulose, Saccharose, Xylit, Sorbit, Mannit, Maltose, Xylose, Glucose, Kaliumchlorid, Natriumchlorid, Polysorbat 80, Polyethylenglykol, Propylenglykol oder Natriumcitrat verwendet werden.

Es kann dabei ein einzelner Porenbildner, aber auch ein Gemisch von wasserunlöslichen oder wasserlöslichen Porenbildnern oder ein Gemisch von wasserlöslichen und wasserunlöslichen Porenbildnern verwendet werden.

Bevorzugt wird ein Porenbildnergemisch von Povidone® K 30, Polysorbat 80 und Eisenoxid rot verwendet.

Die Membranschicht kann man auf verschiedene an sich bekannte Arten auftragen. Günstige Resultate werden besonders durch Aufsprühen einer wässrigen Suspension der Membranschichtbildner auf die vorgeformten Pellets in vorzugsweise einer Wirbelschichtsprühanlage erreicht. Besonders bevorzugt enthält die Wirbelschichtsprühanlage einen Wurster-Einsatz oder einen Rotorprozessor-Einsatz.

Das Gewicht der Membranschicht beträgt vorzugsweise 10-20 Gew.-% der Endpellets. Bevorzugt liegt der Anteil bei etwa 15 +/- 2 Gew.-%, wobei eine Änderung der Menge die Freisetzungscharakteristik wesentlich beeinflusst.

Bevorzugte Ausführungsformen der aufgetragenen Membranschicht enthalten in Gew.-% der Membranschicht z.B.:

| | | | |
|---|---|---|---|
| Eudragit® NE 30 D | 45 % bis 55 %, | vorzugsw. | 52,0-53,5% |
| Povidone® K 30 | 5 % bis 10 %, | vorzugsw. | 8,8-9,8 % |
| Polysorbat 80 | 0,5% bis 1,5%, | vorzugsw. | 1,3 % |
| Eisenoxidrot | 0,5% bis 1,5%, | vorzugsw. | 0,8-1,0 % |
| Talcum | 15 % bis 30 %, | vorzugsw. | 22,0-22,8% |
| Mg-stearat | 10 % bis 15 %, | vorzugsw. | 13,0-13,4% |
| Antischaum-Emulsion SE2 | 0,01% bis 0,1%, | vorzugsw. | 0,05 % |

Eudragit® NE 30 D wird dabei als schichtbildendes wasserunlösliches Copolymer eingesetzt. Die Porenbildner haben die Aufgabe, sich im Darmtrakt aus der Membranschicht herauszulösen oder herauszubrechen, damit die umgebende Flüssigkeit in das Pellet hineindiffundieren und dort Diclofenac auflösen kann. Das gelöste Diclofenac kann dann gemäss der beabsichtigten Freisetzungscharakteristik durch die Poren der Membranschicht in den Magen-/Darmtrakt entlassen werden. Dank dem vordefinierten Aufbau der Membranschicht wird praktisch der gesamte vorhandene Wirkstoff vom Körper resorbiert.

Ein weiterer Vorteil der erfindungsgemässen Membranschicht besteht darin, dass ein Dosierungsintervall der Zubereitung von 12 Stunden genügt, ohne dass es zur schädlichen Kumulation von Wirkstoffkonzentration im Blutplasma kommt und trotzdem eine relativ hohe Anfangskonzentration erreicht werden kann, so dass damit ein frühes Ausfluten des Wirkstoffs in die Synovialflüssigkeit gefördert wird.

In einem weiteren Vorgang wird eine magensaftresistente Lackschicht aufgetragen, die sich oberhalb eines pH-Wertes von 5,5 auflöst. Vorzugsweise wird diese Schicht, die bevorzugt 5-20 Gew.-% der Endpellets, und besonders bevorzugt 10-16 Gew.-%, ausmacht, aufgesprüht. Die magensaftresistente Lackschicht selbst enthält vorzugsweise 60-90 %, besonders bevorzugt 75 Gew.%, mindestens eines säureunlöslichen Polymers und 10-40 %, besonders bevorzugt 25 Gew.-%, Hilfsstoffe.

Als säureunlösliche Polymere kommen z.B. Methacrylsäure-Methacrylsäureester-Copolymere oder Methacrylsäure-Acrylsäureester-Copolymere, wie z.B. Eudragit® L, Eudragit® S, Eudragit® RL 30 D und/oder Eudragit® RS 30 D, oder Aquateric® oder Duodcel® in Frage, und vorzugsweise Eudragit® L 30 D.

Als Hilfsstoffe kommen Weichmacher, wie z.B. Diethylphthalat, Triacetin, Dibutylsebacat, Diethyladipat, Polyethylenglykol oder Tributylcitrat, und Gleit- resp. Trennmittel, wie z.B. Talcum, Syloid®, oder Farbpigmente, wie z.B. Eisenoxid oder Titandioxid, und gegebenenfalls eine Silicon-Antischaumemulsion, wie sie z.B. unter der Bezeichnung SE 2 von der Firma Wacker angeboten wird, in Frage.

Eine bevorzugte Form der magensaftresistenten Lackschicht enthält 70-80 Gew.-% Eudragit® L 30 D, 10-20 Gew.-% Diethylphthalat, 5-15 Gew.-% Talcum und gegebenenfalls 0,1 -0,5 Gew.-% (Trockenanteil 0,02-0,1 Gew.-%), jeweils bezogen auf die Lackschicht, eines Silicon-Antischaummittels.

Die magensaftresistente Lackschicht wird vorzugsweise bis zu einem Anteil von 5-20 Gew.-% der Endpellets, besonders bevorzugt ca. 10-16 Gew.-%, aufgetragen. Falls die Schicht die in USP XXII, S. 1580, festgelegten Kriterien für Magensaftresistenz nicht erfüllt, kann die Lackschicht durch zusätzliches Auftragen weiter verstärkt werden. Die erfindungsgemässe wässrige Suspension für das Auftragen der magensaftresistenten Lackschicht lässt sich ausgezeichnet verarbeiten, wobei das Auftragen an sich auf herkömmliche Art erfolgen kann. Vorzugsweise erfolgt das Aufsprühen der magensaftresistenten Lackschichtbildner jedoch in einer wässrigen Suspension in einer Wirbelschichtsprühanlage mit Wurster-Einsatz oder Rotorprozessor-Einsatz.

Die mit der magensaftresistenten Lackschicht umhüllten Pellets werden zur Entfernung von Agglomeraten und eventuellen Fragmenten gesiebt und klassiert. Die Fraktion zwischen 0,6 mm und 1,4 mm, vorzugsweise 0,8 mm bis 1,25 mm, eignet sich vorzüglich für die Freisetzung von Diclofenac mit den in-vitro-Parametern (USP XXII, S. 1580):
> 15 % nach 1 Stunde
> 30 % nach 2 Stunden
> 50 % nach 3 Stunden
> 70 % nach 4 Stunden
> 80 % nach 5 Stunden.

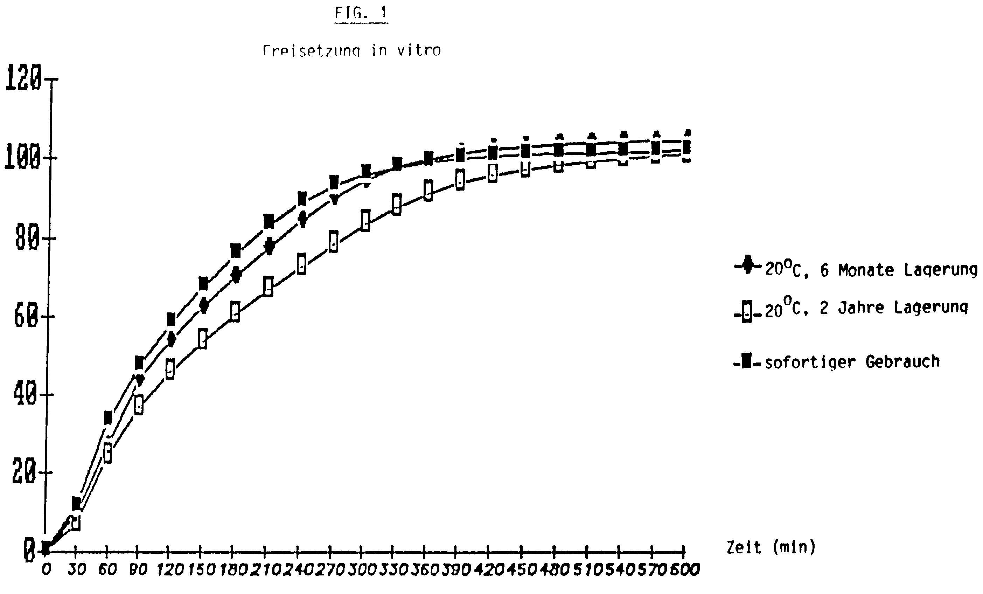

Die erfindungsgemässe Lackschicht weist eine gute Langzeitstabilität auf, so dass auch noch nach 2 Jahren Lagerung die Freisetzungskinematik in-vitro im wesentlichen erhalten bleibt (vgl. Fig.1).

Da die einzelnen Pelletschichten einerseits für sich allein schon mengenmässig in gewissen Grenzen variabel aufgebaut sein können, andererseits die Proportionen der einzelnen Schichten untereinander beim Aufbau des Endpellets variieren können, wurden die Prozentangaben teils auf die einzelnen Schichten und teils auf die Endpellets bezogen.

Im folgenden soll deshalb die art- und mengenmässige Zusammensetzung bevorzugter Endpellets in Gew.-% wiedergegeben werden:

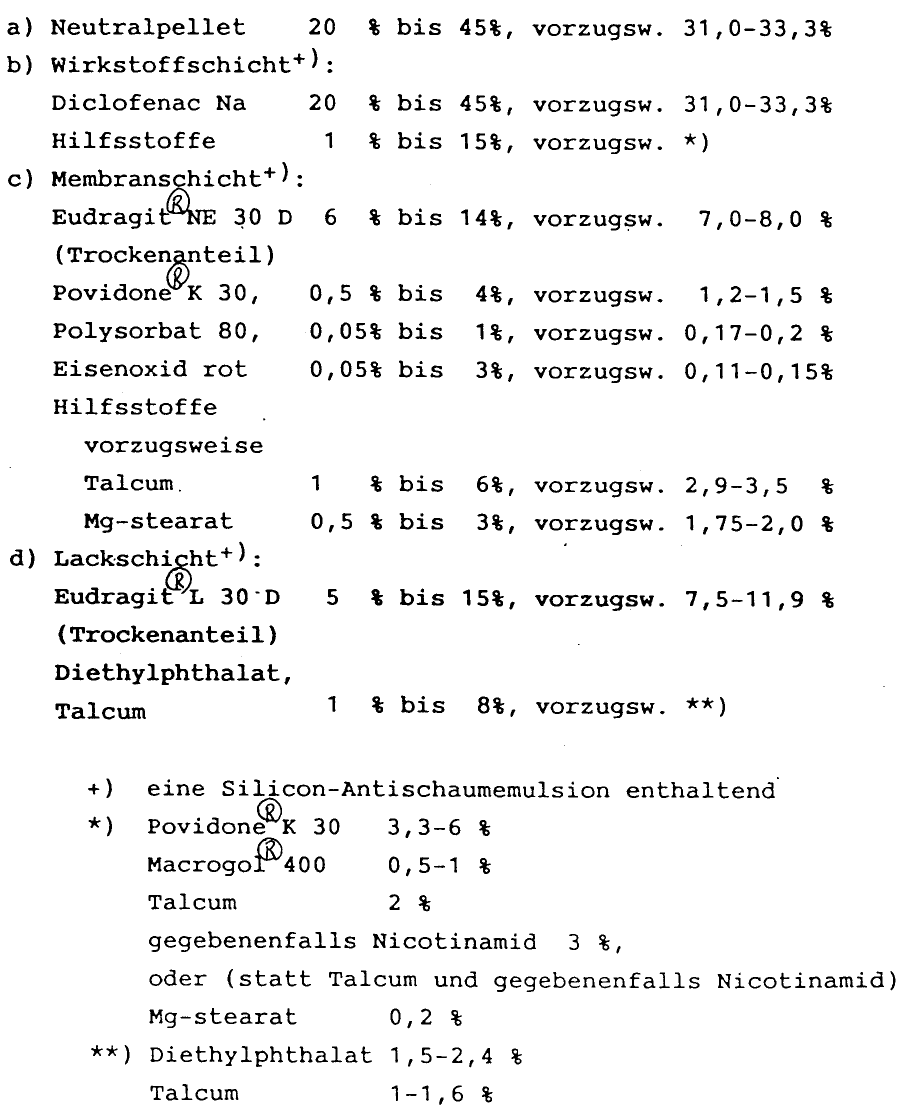

Zwei besonders bevorzugte Formulierungen enthalten:
a) Neutralpellet 31,0 %
b) Wirkstoffschicht⁺⁾: Diclofenac Na 31,0 %, Povidone® K 30 6 %, Macrogol® 400 1 %, Talcum 2 %, Nicotinamid 3 %,
c) Membranschicht⁺⁾: Eudragit® NE 30 D 6 8,0 % (Trockenanteil) , Povidone® K 30 1,5 %, Polysorbat 80 0,2 %, Eisenoxid rot 0,15 %, Talcum 3,5 %, Mg-stearat 2,0 %
d) Lackschicht⁺⁾: Eudragit® L 30 D 7,5 % (Trockenanteil), Diethylphthalat 1,5 %, Talcum 1 %; und
a) Neutralpellet 33,3 %
b) Wirkstoffschicht⁺⁾: Diclofenac Na 33,3 %, Povidone® K 30 3,3 %, Macrogol® 400 0,5 %, Mg-stearat 0,2 %,
c) Membranschicht⁺⁾: Eudragit® NE 30 D 6 7,0 % (Trockenanteil) , Povidone® K 30 1,2 %, Polysorbat® 80 0,17 %, Eisenoxid rot 0,11 %, Talcum 2,9 %, Mg-stearat 1,75 %
d) Lackschicht⁺⁾: Eudragit® L 30 D 11,9 % (Trockenanteil), Diethylphthalat 2,4 %, Talcum 1,6 %;

+) eine Silicon-Antischaumemulsion enthaltend

Vorzugsweise werden die Pellets in Kapseln, besonders bevorzugt in Hartgelatinekapseln, abgefüllt. Therapeutisch ist eine Füllmenge von 50 mg bis 150 mg Diclofenac pro Kapsel, bevorzugt 75 mg (entspricht ca. 155 mg bis 465 mg an Pellets), erforderlich.

Mit der bevorzugten Dosierung von Diclofenac und der unter in-vitro-Bedingungen zu standardisierenden und kontrollierbaren Freisetzungscharakteristik erfolgt nahezu vollständige Resorption des Wirkstoffs im Körper. Die maximalen Blutplasmawerte liegen im Bereich von 300 ng/ml und das Wirkstoffplateau wird wegen der retardierten Wirkstofffreigabe während mehrerer Stunden im Serum aufrechterhalten.

Selbst wenn während längerer Zeit alle 12 Stunden eine erfindungsgemässe Kapsel mit z.B. 75 mg Diclofenac verabreicht wird, kann keine Kumulation der Blutplasmawerte beobachtet werden, weil die Maximalkonzentrationen jeweils innerhalb eines sehr engen Zeitraums erreicht werden. Auch ist die Streuung der gemessenen Blutplasmawerte offensichtlich geringer als nach Verabreichung von Zubereitungen nach dem Stand der Technik.

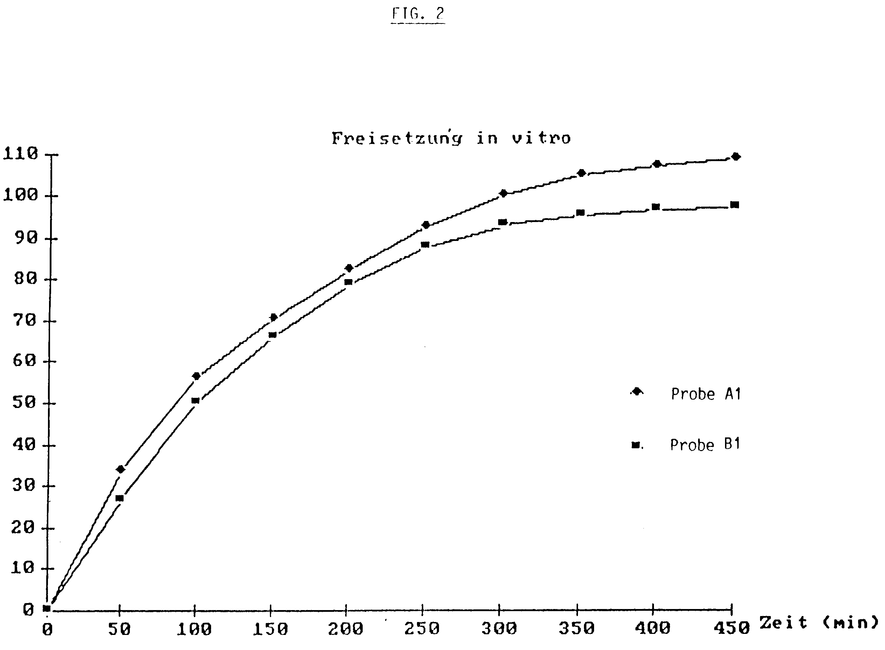

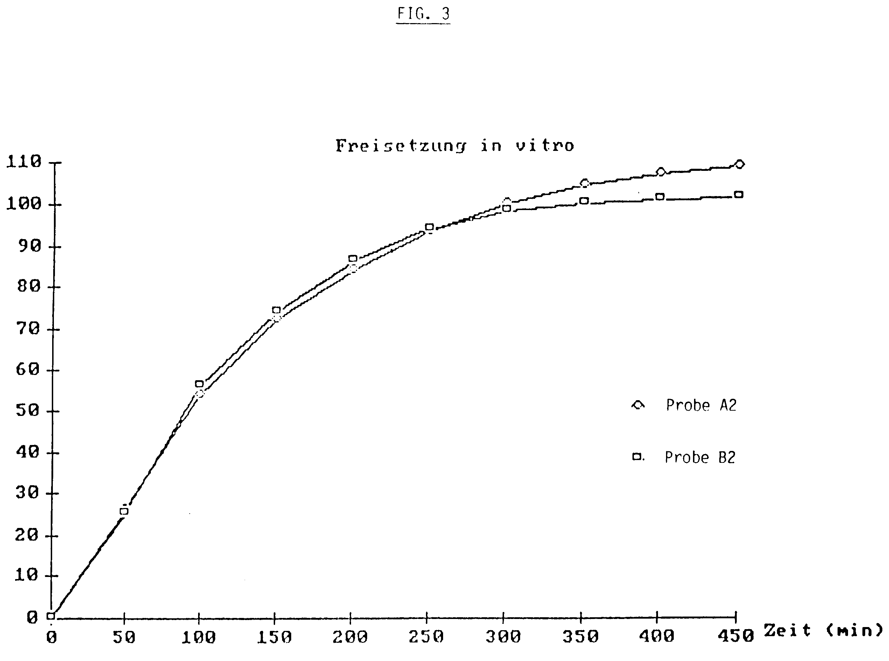

Ein weiterer Vorteil der Erfindung besteht darin, dass es für die Freisetzung von Diclofenac - im Gegensatz zum Stand der Technik - nicht darauf ankommt, ob die Zubereitung einer zweistündigen in-vitro Magensaft-Vorbehandlung unterworfen worden ist. Dies wird aus Fig.2 und Fig.3 ersichtlich, wo die Mittelwerte der Freisetzung im künstlichen Darmsaft von 2 verschiedenen Herstellungschargen einer 75 mg enthaltenden erfindungsgemässen Diclofenaczubereitung einmal mit (Fig.2, Probe A1 und Probe B1) und einmal ohne (Fig.3, Probe A2 und Probe B2) zweistündiger Magensaftvorbehandlung gemessen wurden.

Dieser in-vitro-Versuch wurde an die US-Paddle-Methodik angelehnt und mit dem Gerät SOTAX AT6 nach USP XXI durchgeführt. Die mittlere Dissolutionszeit (sie repräsentiert das Zeitintervall bis zur Freisetzung von 63,2 % Wirkstoff) lag dabei unabhängig von der Vorbehandlung bei ca. 2 Stunden.

Die mittlere Verweilzeit (MRT = mean residence time) des Wirkstoffs beträgt in einer bevorzugten Ausführungsform ca. 5,5 Stunden.

Die geringen Standard-Abweichungen und der fehlende Einfluss einer zweistündigen Vorbehandlung mit Magensaft sind ein Beweis für das regelmässige Verhalten der Freisetzung von Diclofenac aus einer erfindungsgemässen Arzneimittelzubereitung unter sehr verschiedenen Bedingungen. Dies führt in vivo zu einer verringerten Streuung der Blutplasma-Konzentrationen, was für die bekanntermassen an sich schon sehr schwankungsreichen Blutplasma-Konzentrationsverläufe von Diclofenac von ausschlaggebender Bedeutung ist.

Ein besonderer Vorteil der erfindungsgemässen Zubereitung besteht deshalb darin, dass mit der täglichen Verabreichung einer erfindungsgemässen 2 x 75 mg Zubereitung vergleichbare Resultate wie mit einer 3 x 50 mg Standard-Dosierung und bessere Resultate als mit 2 x 100 mg Retarddosierungen gemäss dem Stand der Technik erzielt werden können.

Die Herstellung der beschichteten Pellets kann nach herkömmlichen Verfahren erfolgen, vorzugsweise wird dazu jedoch das Wirbelschichtsprühverfahren benützt. Durch Anwendung dieses Verfahrens und Verwendung der beschriebenen Hilfsmittel brauchen keine organischen Lösungsmittel verwendet werden, so dass auch keine Restlösemittel im Endprodukt anfallen können. Zudem kann durch das unkomplizierte und ökonomische Herstellungsverfahren ein Höchstmass an Reproduzierbarkeit der Freisetzung erzielt, und auf Grund der einheitlich aufgebauten Pellets eine sichere Dosierung bei der Abfüllung in Kapseln sichergestellt werden.

Im folgenden wird die Erfindung anhand von Beispielen näher erläutert:

### Beispiel 1

### Herstellungsformel für 256 kg Pellets

| | |
|---|---|
| Neutralpellets | 80 kg |

| Wirkstoffschicht (Suspension A) | |
|---|---|
| Aqua purificata | 240 kg |
| Silicon-Antischaumemulsion mit einem Trockenanteil von 0.13 kg | 0,65 kg |
| Diclofenac Natrium | 80 kg |
| Nicotinamid | 8 kg |
| Povidone® 30 | 16 kg |
| Macrogol® 400 | 2,4 kg |
| Talcum | 4,8 kg |

| Membranschicht (Suspension B) | |
|---|---|
| Aqua purificata | 53 kg |
| Eudragit® NE 30 D mit einem Trockenanteil von 21 kg | 70 kg |
| Polysorbat 80 | 0,5 kg |
| Povidone® mit K-Value 30 | 3,5 kg |
| Eisenoxid rot E 172 (CI 77491) | 0,35 kg |
| Talcum | 9 kg |
| Magnesium stearat | 5 kg |
| Silicon-Antischaumemulsion mit einem Trockenanteil von 0.02 kg | 0,1 kg |

| Lackschicht (Suspension C) | |
|---|---|
| Aqua purificata | 53 kg |
| Eudragit® L 30 D mit einem Trockenanteil von 19.2 kg | 64 kg |
| Diethylphthalat | 3,8 kg |
| Talcum | 2,7 kg |
| Silicon-Antischaumemulsion mit einem Trockenanteil von 0.02 kg | 0,1 kg |

In einer Wirbelschichtsprühanlage mit Wurster-Einsatz werden die Neutralpellets vorgelegt und mit der Suspension A besprüht. Die Bedingungen sind abhängig vom Gerätetyp und vom Fortschritt des Sprühprozesses.

Anschliessend an die Aufbringung der Wirkstoffschicht in einer Wirbelschichtsprühanlage mit Wurster-Einsatz werden die Pellets mit Suspension B und mit Suspension C besprüht.

Bei Suspension A beträgt die Ablufttemperatur max. 50° C, bei Suspension B und C max. 40° C. Die rel. Feuchtigkeit (hygro-control aw2 Stankowitz, Diepholz, Deutschland) beträgt 25 bis 40 Gew.-%. Sieben und Klassieren erfolgt über Netzsiebe 0,8 mm und 1,4 mm. Korngrössenverteilung (Siebanalyse) liegt typisch im Bereich: < 0,2 Gew.-% < 0,8 mm und < 0,2 Gew.-% > 1,4 mm. Die Ausbeute liegt bei 90-98 Gew.-%.

### Beispiel 2

### Herstellungsformel für 199,5 kg Pellets

| | |
|---|---|
| Neutralpellets | 66,6 kg |

| Wirkstoffschicht (Suspension A) | |
|---|---|
| Aqua purificata | 190 kg |
| Silicon-Antischaumemulsion mit einem Trockenanteil von 0.13 kg | 0,65 kg |
| Diclofenac Natrium | 66,6 kg |
| Povidone® 30 | 6,6 kg |
| Macrogol® 400 | 1,0 kg |
| Mg-stearat | 0,4 kg |

| Membranschicht (Suspension B) | |
|---|---|
| Aqua purificata | 25 kg |
| Eudragit® NE 30 D mit einem Trockenanteil von 14 kg | 47 kg |
| Polysorbat 80 | 0,34 kg |
| Povidone® mit K-Value 30 | 2,4 kg |
| Eisenoxid rot E 172 (CI 77491) | 0,22 kg |
| Talcum | 5,8 kg |
| Magnesium stearat | 3,5 kg |
| Silicon-Antischaumemulsion mit einem Trockenanteil von 0.02 kg | 0,1 kg |

| Lackschicht (Suspension C) | |
|---|---|
| Aqua purificata | 25 kg |
| Eudragit® L 30 D mit einem Trockenanteil von 23,8 kg | 79 kg |
| Diethylphthalat | 4,8 kg |
| Talcum | 3,2 kg |
| Silicon-Antischaumemulsion mit einem Trockenanteil von 0.02 kg | 0,1 kg |

Das Herstellungsverfahren wird analog Bsp. 1 durchgeführt.

### Beispiel 3

### Herstellungsformel für 205 kg Pellets

| | |
|---|---|
| Neutralpellets | 80 kg |

| Wirkstoffschicht (Suspension A) | |
|---|---|
| Aqua purificata | 209 kg |
| Diclofenac Natrium | 80 kg |
| Hydroxypropylmethylcellulose | 8 kg |
| Syloid® 244 FP | 2 kg |
| Silicon-Antischaumemulsion mit einem Trockenanteil von 0,1 kg | 0,5 kg |

| Membranschicht (Suspension B) | |
|---|---|
| Aqua purificata | 30 kg |
| Eudragit® NE 30 D mit einem Trockenanteil von 9,0 kg | 30 kg |
| Talcum | 8 kg |
| Silicon Antischaumemulsion mit einem Trockenanteil von 0,02 kg | 0.1 kg |

| Lackschicht (Suspension C) | |
|---|---|
| Aqua purificata | 26 kg |
| Eudragit® L 30 D mit einem Trockenanteil von 12 kg | 40 kg |
| Dibutylsebacat | 2,5 kg |
| Talcum | 3,5 kg |
| Silicon Antischaumemulsion mit einem Trockenanteil von 0,02 kg | 0,1 kg |

Das Herstellungsverfahren wird analog Bsp. 1 durchgeführt.

### Beispiel 4

### Herstellungsformel für 219 kg Pellets

| | |
|---|---|
| Neutralpellets | 80 kg |

| Wirkstoffschicht (Suspension A) | |
|---|---|
| Aqua purificata | 230 kg |
| Diclofenac Natrium | 80 kg |
| Hydroxy propyl cellulose | 10 kg |
| Magnesiumstearat | 1 kg |
| Nicotinamid | 8 kg |
| Silicon Antischaumemulsion mit einem Trockenanteil von 0,02 kg | 0,1 kg |

| Membranschicht (Suspension B) | |
|---|---|
| Aqua purificata | 26 kg |
| Eudragit® RL 30 D mit einem Trockenanteil von 3,0 kg | 10 kg |
| Eudragit® RS 30 D mit einem Trockenanteil von 9,0 kg | 30 kg |
| Triacetin | 2,5 kg |
| Talcum | 3,5 kg |
| Silicon-Antischaumemulsion mit einem Trockenanteil von 0,01 kg | 0,05 kg |

| Lackschicht (Suspension C) | |
|---|---|
| Aqua purificata | 78 kg |
| Aquateric® | 15 kg |
| Diethylphthalat | 5 kg |
| Polysorbat 80 | 0,2 kg |
| Titandioxid | 2 kg |

Das Herstellungsverfahren wird analog Bsp. 1 durchgeführt.

### Beispiel 5

### Herstellungsformel für 134 kg Pellets.

| | |
|---|---|
| Neutralpellets | 40,0 kg |

| Wirkstoffschicht (Suspension A) | |
|---|---|
| Aqua purificata | 90,0 kg |
| Diclofenac Natrium | 40,0 kg |
| Nicotinamid | 4,0 kg |
| Povidone® K 30 | 1,0 kg |
| Talcum | 2,0 kg |
| Silicon Antischaumemulsion mit Trockenanteil von 0,06 kg | 0,3 kg |

| Membranschicht (Suspension B) | |
|---|---|
| Aqua purificata | 38,0 kg |
| Eudragit® NE 30 D mit Trockenanteil von 15,3 kg | 51,0 kg |
| Eisenoxid rot | 0,25 kg |
| Polysorbat 80 | 0,35 kg |
| Talcum | 6,5 kg |
| Magnesiumstearat | 3,5 kg |
| Hydroxypropylmethylcellulose | 2,5 kg |
| Silicon Antischaumemulsion mit Trockenanteil von 0,02 kg | 0,1 kg |

| Lackschicht (Suspension C) | |
|---|---|
| Aqua purificata | 38,0 kg |
| Eudragit® L 30 D mit Trockenanteil von 13,8 kg | 46,0 kg |
| Diethylphthalat | 2,8 kg |
| Talcum | 2,0 kg |
| Silicon Antischaumemulsion mit Trockenanteil von 0,02 kg | 0,1 kg |

Das Herstellungsverfahren wird analog Beispiel 1 durchgeführt mit den folgenden Ausnahmen:

In einer Wirbelschichtsprühanlage mit Rotorprozessor-Einsatz werden die Neutralpellets vorgelegt und mit Suspension A 5 Min. mit einer Sprührate von 200 g/Min besprüht. Anschliessend wird der Innenzylinder auf eine Spalthöhe von 10 mm geöffnet. Die Prozessluft wird auf 1000 bis 1500 m³/Std eingestellt. Mit fortschreitendem Sprühprozess kann die Sprührate kontinuierlich bis auf 400-500 g/Min gesteigert werden.

Anschliessend an die Aufbringung der Wirkstoffschicht in einem Rotorprozessor werden die Pellets mit Suspension B und C besprüht.

Die Ablufttemperatur liegt bei allen 3 Sprühsuspensionen bei 30-40 °C. Die Scheibe dreht mit 500-900 U/Min.

## Patentansprüche

1. Eine Diclofenac oder eines seiner Salze enthaltende pelletierte orale Arzneimittelzubereitung mit gesteuerter Wirkstofffreisetzung, gekennzeichnet durch eine auf einem Neutralpellet aufgebaute Wirkstoffschicht, eine innere Membranschicht, die 35-65 Gew.-% eines wasserunlöslichen Polymers, 5-20 Gew.-% eines Porenbildners und 20-50 Gew.% Hilfsstoffe enthält, und eine äussere magensaftresistente Lackschicht.

2. Arzneimittelzubereitung nach Patentanspruch 1, dadurch gekennzeichnet, dass die innere Membranschicht 10-20 Gew.-% der Endpellets ausmacht und vorzugsweise 45-55 Gew.-% eines wasserunlöslichen Polymers, 6-13 Gew.-% eines Porenbildners und 25-46 Gew.% Hilfsstoffe enthält.

3. Arzneimittelzubereitung nach einem der Patentansprüche 1 oder 2, dadurch gekennzeichnet, dass die Wirkstoffschicht 20-45 Gew.-% Wirkstoff und 1-15 Gew.-% Hilfsstoffe, bezogen auf die Endpellets, enthält.

4. Arzneimittelzubereitung nach einem der Patentansprüche 1-3, dadurch gekennzeichnet, dass die Wirkstoffschicht als Hilfsstoffe Bindemittel, vorzugsweise Povidone® K 30, oder ein Cellulosederivat, wie Hydroxypropylcellulose oder Hydroxypropylmethylcellulose, Gleit- resp. Trennmittel, wie Mg-stearat, Aerosil® und/oder Syloid® 244 FP oder vorzugsweise Talcum, gegebenenfalls einen Weichmacher, vorzugsweise Macrogol® 400, gegebenenfalls ein Silicon-Antischaummittel und gegebenenfalls einen Indikator, wie Nicotinamid, enthält.

5. Arzneimittelzubereitung nach einem der Patentansprüche 1-4, dadurch gekennzeichnet, dass die Membranschicht als wasserunlösliches Polymer Eudragit® RL 30 D, Eudragit® RS 30 D, Eudragit® NE 30 D, Surelease® und/oder Surelease® XM enthält.

6. Arzneimittelzubereitung nach einem der Patentansprüche 1-5, dadurch gekennzeichnet, dass die Membranschicht als Porenbildner wasserunlösliche Stoffe, oder wasserlösliche Stoffe, oder ein Gemisch aus wasserlöslichen und wasserunlöslichen Stoffen enthält.

7. Arzneimittelzubereitung nach Patentanspruch 6, dadurch gekennzeichnet, dass als wasserunlösliche Stoffe Kaolin, Calciumcarbonat, Calciumhydrogenphosphat, Magnesiumoxid, mikrokristalline Cellulose, Titandioxid und/oder Eisenoxid verwendet werden.

8. Arzneimittelzubereitung nach Patentanspruch 6, dadurch gekennzeichnet, dass als wasserlösliche Stoffe Povidone®, Polyvinylalkohol, Cellulosederivate, wie Hydroxypropylcellulose, Hydroxypropylmethylcellulose, Methylcellulose oder Natriumcarboxymethylcellulose, Polysorbat 80, Saccharose, Xylit, Sorbit, Mannit, Maltose, Xylose, Glucose, Kaliumchlorid, Natriumchlorid, Polyethylenglykol, Propylenglykol und/oder Natriumcitrat verwendet werden.

9. Arzneimittelzubereitung nach einem der Patentansprüche 1-8, dadurch gekennzeichnet, dass die Membranschicht als Hilfsstoffe Weichmacher, wie Triacetin oder Diethylphthalat, und/oder Gleitmittel, wie Talcum, Mg-stearat, Syloid® oder Aerosil®, und gegebenenfalls ein Silicon-Antischaummittel enthält.

10. Arzneimittelzubereitung nach einem der Patentansprüche 1-9, dadurch gekennzeichnet, dass die Membranschicht 45-55 Gew.-% Eudragit® NE 30 D, 5-10 Gew.-% Povidone® K 30, 0,5-1,5 Gew.-% Polysorbat 80, 0,5-1,5 Gew.-% Eisenoxid rot, 15-30 Gew.-% Talcum, 10-15 Gew.-% Mg-stearat, und gegebenenfalls 0,01-0,1 Gew.-%, jeweils bezogen auf die Membranschicht, Silicon-Antischaummittel enthält.

11. Arzneimittelzubereitung nach einem der Patentansprüche 1-10, dadurch gekennzeichnet, dass die magensaftresistente Lackschicht 5-20 Gew.-%, bezogen auf die Endpellets, vorzugsweise 10-16 Gew.-%, beträgt und 60-90 Gew.-%, vorzugsweise 75 Gew.-%, der magensaftresistenten Lackschicht aus einem säureunlöslichen Polymer und 10-40 Gew.-%, vorzugsweise 25 Gew.-%, jeweils bezogen auf die Lackschicht, aus Hilfsstoffen besteht.

12. Arzneimittelzubereitung nach einem der Patentansprüche 1-11, dadurch gekennzeichnet, dass die magensaftresistente Lackschicht ein Methacrylsäurepolymer, wie Eudragit® L 30 D, Eudragit® RL 30 D und/oder Eudragit® RS 30 D, oder Cellulosederivate, wie Aquateric® und/oder Duodcel®, Weichmacher, wie Diethylphthalat, Triacetin, Dibutylsebacat, Diethyladipat, Polyethylenglykol und/oder Tributylcitrat, Gleit- resp. Trennmittel, wie Talcum und/oder eine Kieselsäure, wie Syloid®, gegebenenfalls Farbpigmente, wie Eisenoxid und/oder Titandioxid, und/oder gegebenenfalls ein Silicon-Antischaummittel enthält.

13. Arzneimittelzubereitung nach einem der Patentansprüche 1-12, dadurch gekennzeichnet, dass die magensaftresistente Lackschicht 5-20 Gew.-%, vorzugsweise 10-16 Gew.-%, bezogen auf die Endpellets ausmacht, und 70-80 Gew.-%, insbesondere 75 Gew.-%, Eudragit® L 30 D, 10-20 Gew.-%, insbesondere 15 Gew.-%, Diethylphthalat, 5-15 Gew.-%, insbesondere 10 Gew. -%, Talcum und gegebenenfalls ein Silicon-Antischaummittel enthält.

14. Arzneimittelzubereitung nach einem der Patentansprüche 1-13, dadurch gekennzeichnet, dass das Neutralpellet 20-45 Gew.-% der Endpellets beträgt, die Wirkstoffschicht 20-45 Gew.-% Diclofenac und 1-15 Gew.-% Hilfsstoffe, jeweils bezogen auf die Endpellets, die Membranschicht 6-14 Gew.-% Eudragit® NE 30 D (Trockenanteil), 0,5-4 Gew.-% Povidone® K 30, 0,05-1 Gew.-% Polysorbat 80, 0,05-3 Gew.-% Eisenoxid rot, 1-6 Gew.-% Talcum, und 0,5-3 Gew.-% Mg-stearat, jeweils bezogen auf die Endpellets, und die magensaftresistente Lackschicht 5-15 Gew.-% Eudragit® L 30 D (Trockenanteil) und 1-8 Gew.-%, jeweils bezogen auf die Endpellets, Diethylphthalat und Talcum enthält.

15. Arzneimittelzubereitung nach einem der Patentansprüche 1-14, dadurch gekennzeichnet, dass das Neutralpellet 31 Gew.-% der Endpellets beträgt, die Wirkstoffschicht 31 Gew.-% Diclofenac, 6 Gew.-% Povidone® K 30, 1 Gew.-% Macrogol® 400, 2 Gew.-% Talcum, und gegebenenfalls ein Silicon-Antischaummittel und 3 Gew.-% Nicotinamid, die Membranschicht 8 Gew.-% Eudragit® NE 30 D (Trockenanteil), 0,2 Gew.-% Polysorbat 80, 1,5 Gew.-% Povidone® K 30, 0,15 Gew.-% Eisenoxid rot, 3,5 Gew.-% Talcum, 2 Gew.-% Magnesiumstearat, und gegebenenfalls ein Silicon-Antischaummittel, und die Lackschicht 7,5 Gew.-% Eudragit® L 30 D (Trockenanteil), 1,5 Gew.-% Diethylphthalat und 1 Gew.-%, jeweils bezogen auf die Endpellets, Talcum, und gegebenenfalls ein Silicon-Antischaummittel, enthält.

16. Arzneimittelzubereitung nach einem der Patentansprüche 1-14, dadurch gekennzeichnet, dass das Neutralpellet 33,3 Gew.-% der Endpellets beträgt, die Wirkstoffschicht 33,3 Gew.-% Diclofenac, 3,3 Gew.-% Povidone® K 30, 0,5 Gew.-% Macrogol® 400, 0,2 Gew.-% Mg-stearat, und gegebenenfalls ein Silicon-Antischaummittel, die Membranschicht 7 Gew.-% Eudragit® NE 30 D (Trockenanteil), 0,17 Gew.-% Polysorbat 80, 1,2 Gew.-% Povidone® K 30, 0,11 Gew.-% Eisenoxid rot, 2,9 Gew.-% Talcum, 1,75 Gew.-% Mg-stearat, und gegebenenfalls ein Silicon-Antischaummittel, und die Lackschicht 11,9 Gew.-% Eudragit® L 30 D (Trockenanteil), 2,4 Gew.-% Diethylphthalat und 1,6 Gew.-%, jeweils bezogen auf die Endpellets, Talcum, und gegebenenfalls ein Silicon-Antischaummittel, enthält.

17. Eine Diclofenac oder eines seiner Salze enthaltende pelletierte orale Arzneimittelzubereitung mit gesteuerter Wirkstofffreisetzung nach einem der Patentansprüche 1-16, gekennzeichnet durch eine annähernde in-vitro-Freisetzungsrate von >15 % Wirkstoff nach 1 Stunde, >30 % nach 2 Stunden, >50 % nach 3 Stunden, >70 % nach 4 Stunden und >80 % nach 5 Stunden.

18. Kapsel, insbesondere Hartgelatinekapsel, enthaltend 75 mg Diclofenac oder eines seiner Salze in Form von Pellets gemäss einem der Patentansprüche 1-17.

19. Verfahren zur Herstellung einer Diclofenac oder eines seiner Salze enthaltenden Arzneimittelzubereitung mit gesteuerter Wirkstofffreisetzung nach einem der Patentansprüche 1-17, dadurch gekennzeichnet, dass man in einem ersten Verfahrensschritt die Wirkstoffschicht durch Besprühen von Neutralpellets mittels einer den Wirkstoff enthaltenden Suspension bildet, in einem zweiten Verfahrensschritt die Membranschichtbildner auf die Wirkstoffschicht und in einem dritten Verfahrensschritt die suspendierten magensaftresistenten Lackschichtbildner auf die Membranschicht aufsprüht, die Pellets trocknet und dann diejenigen der geeigneten Korngrösse von >o,8 mm und <1,4 mm aussiebt.

20. Verfahren nach Patentanspruch 19, dadurch gekennzeichnet, dass das Verfahren in einer Wirbelschichtsprühanlage, die vorzugsweise einen Wurster-Einsatz aufweist, durchgeführt wird.

21. Verfahren nach Patentanspruch 19, dadurch gekennzeichnet, dass das Verfahren in einer Wirbelschichtsprühanlage, die einen Rotorprozessor-Einsatz aufweist, durchgeführt wird.

22. Verfahren nach einem der Patentansprüche 19-21, dadurch gekennzeichnet, dass man als Lösungs- resp. Suspensionsmittel Wasser einsetzt.

## Claims

1. A pelleted oral drug preparation containing diclofenac or one of its salts, with controlled release of the active ingredient, which comprises an active ingredient layer built up on an inert pellet, an inner membrane layer containing 35-65% by weight of a water-insoluble polymer, 5-20% by weight of a pore-forming agent and 20-50% by weight of adjuncts, and an outer film coating resistant to gastric juice.

2. A drug preparation according to claim 1 wherein the inner membrane layer makes up 10-20% by weight of the final pellets and preferably contains 45-55% by weight of a water-insoluble polymer, 6-13% by weight of a pore-forming agent and 25-46% by weight of adjuncts.

3. A drug preparation according to claim 1 or claim 2 wherein the active ingredient layer contains 20-45% by weight of active ingredient and 1-15% by weight of adjuncts, based on the final pellets.

4. A drug preparation according to any one of claims 1-3 wherein the active ingredient layer contains, as adjuncts, binders, preferably povidone® K 30 or a cellulose derivative such as hydroxypropyl cellulose or hydroxypropyl methyl cellulose, lubricants and/or release agents such as magnesium stearate, Aerosil® and/or Syloid® 244 FP or, preferably, talc, if necessary a plasticizer, preferably macrogol® 400, if necessary a silicone antifoam and if necessary an indicator such as nicotinamide.

5. A drug preparation according to any one of claims 1-4 wherein the membrane layer contains Eudragit® RL 30 D, Eudragit® RS 30 D, Eudragit® NE 30 D, Surelease® and/or Surelease® XM as the water-insoluble polymer.

6. A drug preparation according to any one of claims 1-5 wherein the membrane layer contains water-insoluble substances, or water-soluble substances, or a mixture of water-soluble and water-insoluble substances as pore-forming agents.

7. A drug preparation according to claim 6 wherein kaolin, calcium carbonate, calcium hydrogen phosphate, magnesium oxide, microcrystalline cellulose, titanium dioxide and/or iron oxide are used as water-insoluble substances.

8. A drug preparation according to claim 6 wherein povidone®, polyvinyl alcohol, cellulose derivatives such as hydroxypropyl cellulose, hydroxypropyl methyl cellulose, methyl cellulose or sodium carboxymethyl cellulose, polysorbate 80, sucrose, xylitol, sorbitol, mannitol, maltose, xylose, glucose, potassium chloride, sodium chloride, polyethylene glycol, propylene glycol and/or sodium citrate are used as water-soluble substances.

9. A drug preparation according to any one of claims 1-8 wherein the membrane layer contains, as adjuncts, plasticizers such as triacetin or diethyl phthalate, and/or lubricants such as talc, magnesium stearate, Syloid® or Aerosil®, and if necessary a silicone antifoam.

10. A drug preparation according to any one of claims 1-9 wherein the membrane layer contains 45-55% by weight of Eudragit® NE 30 D, 5-10% by weight of povidone® K 30, 0.5-1.5% by weight of polysorbate 80, 0.5-1.5% by weight of red iron oxide, 15-30% by weight of talc, 10-15% by weight of magnesium stearate and if necessary 0.01-0.1% by weight of silicone antifoam, based in each case on the membrane layer.

11. A drug preparation according to any one of claims 1-10 wherein the film coating resistant to gastric juice makes up 5-20% by weight, preferably 10-16% by weight, based on the final pellets, and 60-90% by weight, preferably 75% by weight, of the film coating resistant to gastric juice consists of an acid-insoluble polymer and 10-40% by weight, preferably 25% by weight, consists of adjuncts, based in each case on the film coating.

12. A drug preparation according to any one of claims 1-11 wherein the film coating resistant to gastric juice contains a methacrylic acid polymer such as Eudragit® L 30 D, Eudragit® RL 30 D and/or Eudragit® RS 30 D, or cellulose derivatives such as Aquateric® and/or Duodcel®, plasticizers such as diethyl phthalate, triacetin, dibutyl sebacate, diethyl adipate, polyethylene glycol and/or tributyl citrate, lubricants and/or release agents such as talc and/or a silicic acid like Syloid®, if necessary colored pigments such as iron oxide and/or titanium dioxide, and/or if necessary a silicone antifoam.

13. A drug preparation according to any one of claims 1-12 wherein the film coating resistant to gastric juice makes up 5-20% by weight, preferably 10-16% by weight, based on the final pellets, and contains 70-80% by weight, especially 75% by weight, of Eudragit® L 30 D, 10-20% by weight, especially 15% by weight, of diethyl phthalate, 5-15% by weight, especially 10% by weight, of talc and if necessary a silicone antifoam.

14. A drug preparation according to any one of claims 1-13 wherein the inert pellet makes up 20-45% by weight of the final pellets, the active ingredient layer contains 20-45% by weight of diclofenac and 1-15% by weight of adjuvants, based in each case on the final pellets, the membrane layer contains 6-14% by weight of Eudragit® NE 30 D (solids content), 0.5-4% by weight of povidone® K 30, 0.05-1% by weight of polysorbate 80, 0.05-3% by weight of red iron oxide, 1-6% by weight of talc and 0.5-3% by weight of magnesium stearate, based in each case on the final pellets, and the film coating resistant to gastric juice contains 5-15% by weight of Eudragit® L 30 D (solids content) and 1-8% by weight of diethyl phthalate and talc, based in each case on the final pellets.

15. A drug preparation according to any one of claims 1-14 wherein the inert pellet makes up 31% by weight of the final pellets, the active ingredient layer contains 31% by weight of diclofenac, 6% by weight of povidone® K 30, 1% by weight of macrogol® 400, 2% by weight of talc, if necessary a silicone antifoam and 3% by weight of nicotinamide, the membrane layer contains 8% by weight of Eudragit® NE 30 D (solids content), 0.2% by weight of polysorbate 80, 1.5% by weight of povidone® K 30, 0.15% by weight of red iron oxide, 3.5% by weight of talc, 2% by weight of magnesium stearate and if necessary a silicone antifoam, and the film coating contains 7.5% by weight of Eudragit® L 30 D (solids content), 1.5% by weight of diethyl phthalate and 1% by weight of talc, based in each case on the final pellets, and if necessary a silicone antifoam.

16. A drug preparation according to any one of claims 1-14 wherein the inert pellet makes up 33.3% by weight of the final pellets, the active ingredient layer contains 33.3% by weight of diclofenac, 3.3% by weight of povidone® K 30, 0.5% by weight of macrogol® 400, 0.2% by weight of magnesium stearate and if necessary a silicone antifoam, the membrane layer contains 7% by weight of Eudragit® NE 30 D (solids content), 0.17% by weight of polysorbate 80, 1.2% by weight of povidone® K 30, 0.11% by weight of red iron oxide, 2.9% by weight of talc, 1.75% by weight of magnesium stearate and if necessary a silicone antifoam, and the film coating contains 11.9% by weight of Eudragit® L 30 D (solids content), 2.4% by weight of diethyl phthalate and 1.6% by weight of talc, based in each case on the final pellets, and if necessary a silicone antifoam.

17. A pelleted oral drug preparation containing diclofenac or one of its salts, with controlled release of the active ingredient, according to any one of claims 1-16 wherein the approximate *in vitro* release rate is >15% of active ingredient after 1 hour, >30% after 2 hours, >50% after 3 hours, >70% after 4 hours and >80% after 5 hours.

18. A capsule, especially a hard gelatin capsule, containing 75 mg of diclofenac or one of its salts in the form of pellets according to any one of claims 1-17.

19. A process for the manufacture of a drug preparation containing diclofenac or one of its salts, with controlled release of the active ingredient, according to any one of claims 1-17, wherein in a first process step the active ingredient layer is formed by spraying inert pellets with a suspension containing the active ingredient, in a second process step the agents forming the membrane layer are sprayed on to the active ingredient layer, and in a third process step the suspended agents forming the film coating resistant to gastric juice are sprayed on to the membrane layer, the pellets are dried and those with the suitable grain size of >0.8 mm and <1.4 mm are then sieved out.

20. A process according to claim 19 which is carried out in a fluidized-bed spray unit preferably having a Wurster insert.

21. A process according to claim 19 which is carried out in a fluidized-bed spray unit having a rotor processor insert.

22. A process according to any one of claims 19-21 wherein water is used as solvent and/or suspending agent.

## Revendications

1. Préparation pharmaceutique par voie orale, mise sous forme de pellets, contenant du diclofénac ou l'un de ses sels, avec une libération régulée du principe actif, caractérisée par une couche de principe actif constituée sur un pellet neutre, une couche interne formant membrane qui contient 35-65% en poids d'un polymère insoluble dans l'eau, 5-20% en poids d'un agent de formation des pores et 20-50% en poids de substances auxiliaires et par une couche externe d'enrobage résistant au suc gastrique.

2. Préparation pharmaceutique selon la revendication 1, caractérisée en ce que la couche interne formant membrane correspond à 10-20% en poids des pellets finaux et contient de préférence 45-55% en poids d'un polymère insoluble dans l'eau, 6-13% en poids d'un agent de formation des pores et 25-46% en poids de substances auxiliaires.

3. Préparation pharmaceutique selon les revendications 1 ou 2, caractérisée en ce que la couche de principe actif contient 20-45% en poids de principe actif et 1-15% en poids de substances auxiliaires, en référence aux pellets finaux.

4. Préparation pharmaceutique selon l'une quelconque des revendications 1 à 3, caractérisée en ce que la couche de principe actif contient en tant que substances auxiliaires, des liants, de préférence Povidone® K 30 ou un dérivé de cellulose comme l'hydroxypropylcellulose ou l'hydroxypropylméthylcellulose, des lubrifiants et/ou des agents séparateurs comme le stéarate de magnésium, Aerosil® et/ou Syloid® 244 FP ou de préférence de la poudre de talc, le cas échéant un émollient, de préférence Macrogol® 400, le cas échéant, un agent anti-moussant à la silicone et le cas échéant un indicateur comme le nicotinamide.

5. Préparation pharmaceutique selon l'une quelconque des revendications 1 à 4, caractérisée en ce que la couche formant membrane contient en tant que polymère insoluble dans l'eau, Eudragit® RL 30 D, Eudragit® RS 30 D, Eudragit® NE 30 D, Surelease® et/ou Surelease® XM.

6. Préparation pharmaceutique selon l'une quelconque des revendications 1 à 5, caractérisée en ce que la couche formant membrane contient en tant qu'agent de formation des pores, des substances insolubles dans l'eau ou hydrosolubles ou un mélange de substances hydrosolubles et insolubles dans l'eau.

7. Préparation pharmaceutique selon la revendication 6, caractérisée en ce que les substances insolubles dans l'eau utilisées sont le kaolin, le carbonate de calcium, l'hydrophosphate de calcium, l'oxyde de magnésium, la cellulose microcristalline, l'oxyde de titane et/ou l'oxyde de fer.

8. Préparation pharmaceutique selon la revendication 6, caractérisée en ce que les substances hydrosolubles utilisées sont la Povidone®, le polyalcool de vinyle, les dérivés de cellulose comme l'hydroxypropylcellulose, l'hydroxypropylméthylcellulose, la méthylcellulose ou la carboxyméthylcellulose de sodium, Polysorbat 80, la saccharose, la xylite, le sorbitol, le mannitol, le maltose, le xylose, le glucose, le chlorure de potassium, le chlorure de sodium, le polyéthylèneglycol, le propylèneglycol et/ou le citrate de sodium.

9. Préparation pharmaceutique selon l'une quelconque des revendications 1 à 8, caractérisée en ce que la couche formant membrane contient comme substances auxiliaires des émollients comme la triacétine ou le diéthylphtalate et/ou des lubrifiants comme la poudre de talc, le stéarate de magnésium, Syloid® ou Aerosil® et le cas échéant un agent anti-moussant à la silicone.

10. Préparation pharmaceutique selon l'une quelconque des revendications 1 à 9, caractérisée en ce que la couche formant membrane contient 45-55% en poids d'Eudragit® NE 30 D, 5-10% en poids de Povidone® K 30, 0,5-1,5% en poids de Polysorbat 80, 0,5-1,5% en poids d'oxyde de fer rouge, 15-30% en poids de poudre de talc, 10-15% en poids de stéarate de magnésium et le cas échéant 0,01-0,1% en poids d'agent anti-moussant à la silicone respectivement en référence à la couche formant membrane.

11. Préparation pharmaceutique selon l'une quelconque des revendications 1 à 10, caractérisée en ce que la couche d'enrobage résistant au suc gastrique correspond à 5-20% en poids, en référence aux pellets finaux, de préférence à 10-16% en poids, que 60-90% en poids, de préférence 75% en poids de la couche d'enrobage résistant au suc gastrique se composent d'un polymère insoluble dans l'acide et que 10-40% en poids, de préférence 25% en poids se composent de substances auxiliaires, respectivement en référence à la couche d'enrobage.

12. Préparation pharmaceutique selon l'une quelconque des revendications 1 à 11, caractérisée en ce que la couche d'enrobage résistant au suc gastrique contient un polymère méthacrylique comme Eudragit® L 30 D, Eudragit® RL 30 D et/ou Eudragit® RS 30 D ou des dérivés de cellulose comme Aquateric® et/ou Duodcel®, des émollients comme le diéthylphtalate, la triacétine, le dibutylsébacate, le diéthyladipate, le polyéthlèneglycol et/ou le tributylcitrate, des lubrifiants et/ou des agents séparateurs comme la poudre de talc et/ou un acide silicique, comme Syloid®, le cas échéant des pigments colorés comme l'oxyde de fer et/ou l'oxyde de titane et/ou le cas échéant un agent anti-moussant à la silicone.

13. Préparation pharmaceutique selon l'une quelconque des revendications 1 à 12, caractérisée en ce que la couche d'enrobage résistant au suc gastrique correspond à 5-20% en poids, de préférence 10-16% en poids en référence aux pellets finaux, et contient 70-80% en poids, en particulier 75% en poids d'Eudragit® L 30 D, 10-20% en poids, en particulier 15% en poids de diéthylphtalate, 5-15% en poids, en particulier 10% en poids de poudre de talc et le cas échéant un agent anti-moussant à la silicone.

14. Préparation pharmaceutique selon l'une quelconque des revendications 1 à 13, caractérisée en ce que le pellet neutre correspond à 20-45% en poids des pellets finaux, que la couche de principe actif contient 20-45% en poids de diclofénac et 1-15% en poids de substances auxiliaires, respectivement en référence aux pellets finaux, que la couche formant membrane contient 6-14% en poids d'Eudragit® NE 30 D (extrait sec), 0,5-4% en poids de Povidone® K 30, 0,05-1% en poids de polysorbate 80, 0,05-3% en poids d'oxyde de fer rouge, 1-6% en poids de poudre de talc et 0,5-3% en poids de stéarate de magnésium, respectivement en référence aux pellets finaux et que la couche d'enrobage résistante au suc gastrique contient 5-15% d'Eudragit® L 30 D (extrait sec) et 1-8% en poids de diéthylphtalate et de poudre de talc, respectivement en référence aux pellets finaux.

15. Préparation pharmaceutique selon l'une quelconque des revendications 1 à 14, caractérisée en ce que le pellet neutre correspond à 31% en poids des pellets finaux, que la couche de principe actif contient 31% en poids de diclofénac, 6% en poids de Povidone® K 30, 1% en poids de Macrogol® 400, 2% en poids de poudre de talc et le cas échéant un agent anti-moussant à la silicone et 3% en poids de nicotinamide, que la couche formant membrane contient 8 % en poids d'Eudragit® NE 30 D (extrait sec), 0,2% de polysorbate 80, 1,5% en poids de Povidone® K 30, 0,15% en poids d'oxyde de fer rouge, 3,5% en poids de poudre de talc, 2% en poids de stéarate de magnésium et le cas échéant un agent anti-moussant à la silicone et que la couche d'enrobage contient 7,5% en poids d'Eudragit® L 30 D (extrait sec), 1,5% en poids de diéthylphtalate et 1% en poids de poudre de talc, respectivement en référence aux pellets finaux et le cas échéant un agent anti-moussant à la silicone.

16. Préparation pharmaceutique selon l'une quelconque des revendications 1 à 14, caractérisée en ce que le pellet neutre correspond à 33,3% en poids des pellets finaux, que la couche de principe actif contient 33,3% en poids de diclofénac, 3,3% en poids de Povidone® K 30, 0,5% en poids de Macrogol® 400, 0,2% en poids de stéarate de magnésium et le cas échéant un agent anti-moussant à la silicone, que la couche formant membrane contient 7% en poids d'Eudragit® NE 30 D (extrait sec), 0,17% de polysorbate 80, 1,2% en poids de Povidone® K 30, 0,11% en poids d'oxyde de fer rouge, 2,9% en poids de poudre de talc, 1,75% en poids de stéarate de magnésium et le cas échéant un agent anti-moussant à la silicone et que la couche d'enrobage contient 11,9% en poids d'Eudragit® L 30 D (extrait sec), 2,4% en poids de diéthylphtalate et 1,6% en poids de poudre de talc, respectivement en référence aux pellets finaux et le cas échéant un agent anti-moussant à la silicone.

17. Une préparation pharmaceutique par voie orale, mise sous forme de pellets, contenant du diclofénac ou l'un de ses sels, avec une libération régulée du principe actif, selon l'une quelconque des revendications 1 à 16, caractérisée par un taux de libération in vitro approximatif de > 15% du principe actif au bout d'1 heure, de > 30% au bout de 2 heures, de > 50% au bout de 3 heures, de > 70% au bout de 4 heures et de > 80% au bout de 5 heures.

18. Capsule, notamment capsule de gélatine dure, contenant 75 mg de diclofénac ou l'un de ses sels sous forme de pellets selon l'une quelconque des revendications 1 à 17.

19. Procédé de fabrication d'une préparation pharmaceutique contenant du diclofénac ou l'un de ses sels, avec une libération régulée du principe actif, selon l'une quelconque des revendications 1 à 17, caractérisé en ce que dans une première étape de préparation, on forme la couche du principe actif par vaporisation de pellets neutres à l'aide d'une suspension contenant le principe actif, que dans une seconde étape de préparation, on vaporise l'agent de formation de la couche formant membrane sur la couche du principe actif et que dans une troisième étape de préparation, on vaporise sur la couche formant membrane les agents de formation résistant au suc gastrique et en suspension, que l'on sèche les pellets et qu'ensuite on les trie en les tamisant à la grosseur de grain de >0,8 mm et <1,4 mm.

20. Procédé selon la revendication 19, caractérisé en ce que la préparation est effectuée dans un système de vaporisation par lit fluidisé qui présente de préférence un module Wurster.

21. Procédé selon la revendication 19, caractérisé en ce que la préparation est effectuée dans un système de vaporisation par lit fluidisé qui présente de préférence un processeur à rotor .

22. Procédé selon l'une quelconque des revendications 19 à 21, caractérisé en ce que le solvant ou l'agent de suspension utilisé est de l'eau.
